# EUROPEAN PATENT APPLICATION

(11) **EP 2 889 026 A1**
(43) Date of publication of application: **01.07.2015**
(21) Application number: 14199583.7
(22) Date of filing: 22.12.2014
(51) Int. Cl.: A61K 8/31, A61K 8/34, A61K 8/49, A61Q 11/00, A61K 8/97

(54) **Compositions and methods for the prevention and treatment of oral diseases**

(30) Priority: 24.12.2013 US 201314140464
(71) Applicant: Generex Pharmaceuticals, Inc., Hong Kong (CN)
(72) Inventor: Ming, Li, Tuen mun, New territories, Hong Kong Island (HK)
(74) Representative: Jacobi, Markus Alexander

(57) **Abstract**

Product for oral healthcare which includes an extract from a plant species containing chlorophyll, polyphenols and triterpenoids, which is referred to as "aCPT", in a dosage form such as toothpaste or mouth wash, where the extract is prepared by extracting the plant with an organic solvent such as ethanol or methanol.

## Description

### FILED OF THE INVENTION

The present invention relates to an oral healthcare product. Particularly, it relates to products, such as toothpastes, that include an active extract obtained from herbal extracts.

### BACKGROUND OF THE INVENTION

Oral health is important to one's overall health. Reports have shown a close correlation between poor oral health and systemic diseases, such as heart disease, diabetes, and some respiratory diseases. Oral diseases, including tooth decay, mouth ulcers, bleeding gums, gingivitis, periodontitis, gum receding and bone loss, are considered the third largest killer of human beings. Therefore, it is of great importance to develop new methods for effective prevention and treatment of these diseases.

Dental caries (tooth decay) is one of the most prevalent diseases next only to the common cold, with prevalence between 80-90% in children and more than 90% in elderly persons in some countries. Dental caries is an oral disease where the causal bacteria, including mainly Streptococcus mutans and Lactobacillus, have the ability to metabolize carbohydrates in food left on teeth to a weak acid that can demineralize the teeth. As a result, the hard tooth structure progressively breaks down, leading to dental caries.

The gum is the soft tissue surrounding teeth that provides a seal around the teeth, which appears a coral pink color with a firm texture and holds tightly to each tooth. Gum bleeding, also known as bleeding gums or gingival bleeding, is mainly caused by the formation and accumulation of hardened bacterial plaque (tartar) at the gum line due to improper brushing and flossing of teeth. To prevent and stop gum bleeding, it is essential to prevent bacterial growth and deposits on the teeth and the gum.

Dental bone loss refers to bone loss of the teeth and alveolar bone loss, which is mainly caused by gum diseases. Gum recession, a common condition for adults over 40, is often caused by inadequate brushing, flossing, or periodontal diseases, which results in bacterial plaque buildup between the teeth, driving a wedge between the teeth and gums. Although prevention and treatment of gum recession is critical to maintain the oral health, unfortunately, there is no product available to strengthen or regenerate gum tissues and repair receded gums.

### SUMMARY OF THE INVENTION

Accordingly, the present invention provides an oral healthcare composition with an active plant extract containing chlorophyll, polyphenols and triterpenoids as well as other unknown compounds, which are refereed to hereinafter as "aCPT".

The aCPT can be made from many species of Rosaceae family, including fructuo rosae laevigakea (EFRL), Centella asiatica (ECA), unripe fruits of Rubus coreanus (EUFRC), Geum japonicum (GJ) and Rubus imperialis (ERI). Although the extracts from these respective plants contain similar compositions of compounds as compared to each other, the extract obtained from GJ contains more polyphenos. They are all within the meaning of the present invention, and they can be considered as equivalents to each other in use.

These plants have long been used as traditional Chinese medicine. According to the present invention, an extract from these plants (aCPT) is found to be highly effective in treating many of the oral diseases. It was further discovered such aCPT is suitable to be formulated in a number of dosage forms (0.05%-50%) for being conveniently used as daily healthcare products, particularly, in the form of toothpaste. Other suitable dosage forms includes, but not limited by, a cream, an ointment, a gel, a foam, a powder, a spray, a drink, a drink mix, a candy, a lozenge, a mouthwash, a gargle, a gel strip, a dental floss, a lozenge, and a gum.

The aCPT of the plants according to the present invention is prepared by extracting the respective plants, such as EFRL, ECA, EUFRC, GJ and ERI, with a lower alkyl alcohol solvent having 1-6 carbons atoms and water. Preferably, the solvent is ethanol/water or methanol/water. According to the present invention, the aCPT prepared with a lower alkyl alcohol solvent possess potent effects against a number of oral diseases such as, for example, tooth decay, mouth ulcer, swelling and aching of the gum and mouth, oral inflammation, bleeding gums, gingivitis or periodontitis, gum recession, halitosis, dental bone loss, age-related tooth loosening or tooth loss.

As used in the present invention the term "aCPT" means an extract made using the extracting method described below in this application from any plants so long the resulting extract contains chlorophyll, polyphenols and triterpenoids, each at a level comparable to that of rosae laevigakea (EFRL), Centella asiatica (ECA), unripe fruits of Rubus coreanus (EUFRC), Geum japonicum (GJ) and Rubus imperialis (ERI).

### BRIEF DESCRIPTION OF THE DRAWINGS

FIG. 1 shows the prevention of dental bone loss in dKO mice by aCPT treatment.
FIG. 2 shows the GI scores of the mice treated by aCPT made from EFRL, ECA, EUFRC, GJ, and ERI, respectively.
FIG. 3 shows the effect of aCPT in restoration of age-related gum degeneration in old mice.
FIG. 4 shows the protective effect of aCPT on the thickness and dimension of tooth enamels in aged animals.
FIG. 5 shows the inhibition of oral bacterial growth by aCPT. a-e, oral bacterial growth in culture plates containing 5 respective preservatives. aCPT 0.1%, aCPT 0.3%, aCPT 0.5%, oral bacterial growth in culture plates containing 3 respective concentrations of aCPT.
FIG. 6 shows the significant inhibition effect of aCPT on bacterial growth of human oral inflammation origin as compared with the 5 respective preservatives.
FIG. 7 shows the evaluation of plaque index (PLI) after aCPT treatment in human.

The various features of novelty which characterize the invention are pointed out with particularity in the claims annexed to and forming a part of this disclosure. For a better understanding of the invention, its operating advantages, and specific objects attained by its use, reference should be made to the drawings and the following description in which there are illustrated and described preferred embodiments of the invention.

### DETAILED DESCRIPTION OF PARTICULAR EMBODIMENTS OF THE INVENTION

### Preparation of aCPT from plant materials

The method of preparing the extract according to the present invention generally comprises a step (a) of extracting the plant with alcohol selected from the group consisting of C1-C4 alcohols. This step may be repeated 3-6 times, typically 5 times, at room temperature. Before extracting, the plant material may be powdered or cut into small pieces for enhancing the extracting efficiency. The C1-C4 alcohols include methanol, ethanol, n-propanol, iso-propanol, n-butanol, iso-butanol, and ter-butanol. Typically, alcohol is added in 1-10 times by weight of the amount of the dried plant to be extracted. A specific example of the method is described below in detail, which was used to prepare the active extract used to demonstrate its biological effects disclosed wherein.

### Example 1. Isolation of the active extracts from example plants

The EFRL and GJ plants were collected from Taibai County, Shanxi and Guizhou Provinces of China in July. The extracts was made from each of the two plants in the same way detailed below.

The plant was washed, dried (100 kg) and cut into small pieces. The dried pieces of the plant were soaked with 95% ethanol (200 L) at 50°C for 2 hours, to which additional 95% ethanol and water were added to make 60% ethanol (1:10 w/v). The plant pieces were further extracted at 60°C for 6 hours respectively and the resulting extraction was filtered and the plant pieces were further extracted with 60% ethanol (1:10 v/v) at 60°C for 6 hours. The resulting extractions were combined, filtered and electro-spray dried to yield a powder fraction, which was the active extract. Test data showed the active extract contains 20-30% chlorophyll, 50-80% polyphenols, and 1-3% triterpenoids, which referred to as "aCTP" in the present invention. The aCTP was used to formulate a toothpaste, namely aCPT-toothpaste (CPT-T), which was used for all the biological testing disclosed herewith.

It should be readily understandable to a person of ordinary skill in the art, the active extract may be subject to further purification of various degrees using commonly available technology and ordinary skill in the art. Further purification is not necessary for practicing the present invention but may be preferred for some dosage forms as judged by those with ordinary skill in the art. Within the meaning of the present invention, aCPT may be the crude extract prepared with a lower alcohol solvent (as exemplified by the above example 1) with or without further purification as long as it retaining the biological effect according to the present invention.

By NMR analysis, the aCPT of EFRL or GJ made in example 1 was found to contain chlorophyll-a, chlorophyll-b, Casuarinin, 2,3,7,8-tetrahydroxy benzopyrano5,4,3 - cdebenzopyran-5,10 - dione, Gemin A, B, Madecassic acid, Potentillin, Asiatic acid, 2-hydroxyoleanolic acid, Niga-ichigoside F1, Kaji-ichigoside F1, Euscaphic acid, ursolic acid, Asiaticoside, 2-hydroxyoleanolic acid, and 28-β-D-glucoside of tormentic acid. This information should help design of a modified extract and purification procedure if a particular situation requires modification to the method of preparing the active extract. A particular dosage form may require a higher degree of purification than the toothpaste formulation requires.

### Formulations and Dosages of Pharmaceutical Compositions

As a person with ordinary skill in the art understands, the pharmaceutical compositions may comprise pharmaceutically-acceptable carriers are determined in part by the particular composition being administered, as well as by the particular method used to administer the composition. Accordingly, there is a wide variety of suitable formulations of pharmaceutical compositions for administering the compound compositions (see, e.g., Remington's Pharmaceutical Sciences, Mack Publishing Co., Easton, PA 18th ed., 1990).

According to the present invention, the aCPT is suitable to be formulated into a number of dosage forms, for example, a cream, an ointment, a gel, a foam, a powder, a solution, a spray, a candy, a lozenge, a mouthwash, a toothpaste, a gargle, and a gum, mouth wash, mouth rinse, toothpaste, oral care gel strips, and dental floss. Methods of preparing those dosage forms are known in the art and it is not part of the present invention. These formulated dosage forms contain different concentrations (from 0.1% -50% or higher) of aCPT according to the applications for treatment of oral diseases, maintaining oral health or prevention of oral diseases. For instances, when we tested the therapeutic or prevention effects of aCPT on different oral conditions, such as tooth caries, oral bacterial plaque, gum bleeding, periodontitis, gingivitis, loose teeth, mouth ulcer, teeth lost due to aging, gum recession, healing of mouth injuries and etc, the toothpaste containing 0.1-1% aCPT is normally used in normal healthy subjects for prevention of tooth caries, oral bacterial plaque, bad-breath, and its antimicrobial effect allows the toothpaste without the need of use of preservatives; the toothpaste containing 1-3% aCPT is suitable for people with mild to moderate oral problems and prevention of common oral problems with frequent use; the toothpaste containing 4-30% or higher aCPT is suitable for people with moderate to severe oral problems and prevention and improvement of most oral problems with frequent use.

For the embodiments described in this application, the exemplary toothpase was made according to the following formulation:

| | |
|---|---|
| GJ extraction (aCPT) | 3.0% |
| Vitamin B2 | 0.1% |
| Sorbitol | 50.0 % |
| Silicon Dioxide | 30.0% |
| Water | 13.7% |
| Sodium Lauryl Sulfate | 2.2% |
| Menthol, Orange Oil, Lemon Essential Oil | 1.0% |

### Biological Activity of aCPT

Example 2. Therapeutic effects of the aCPT of GJ on experimental periodontitis and inflammatory bone absorption in aging Alzheimer mice (conditional PS1/PS2 double knock-out Alzheimer mice, dKO)

Presenilin 1 (PS1) and presenilin 2 (PS2) are transmembrane proteins that are integral components of γ-secretase, a complex that is responsible for the intramembranous cleavage of the amyloid precursor protein (APP) and the Notch receptors (De Strooper et al., 1998, 1999). Previous reports showed that mutations of either PS1 or PS2 are likely associated with the early onset of Alzheimer disease (AD) (Haass, 1997; Price & Sisodia, 1998). Further studies demonstrated that the mice with both PS1 and PS2 genes conditionally knocked out in the forebrain (dKO mice) exhibit AD-like mild neurodegenerative phenotypes as early as 2 months of age. Severe memory impairment is observed in the dKO mice of 6-month-old. Interestingly, a recent study found that inflammation in the dKO brain could expand to the periphery organs and tissues, including oral tissues, such as abnormalities in the periodontal tissue, oral inflammation and bone absorption (Han et al., 2011). Therefore, dKO AD mice model can serve as a suitable animal model for developing potential treatment for oral diseases, especially those related to aging.

Sixteen dKO mice (6 months old) weighing 20-50 grams were divided into test and control groups. The mice in both groups had free access to water and to rat pellet chow containing 0.5% aCPT obtained from GJ extract for test group and containing no aCPT for control group for two months. On day 61, the animals were sacrificed. The mandibles were removed, decalcified and processed histologically. Tissue sections were stained with H&E and histomorphometric parameters were evaluated.

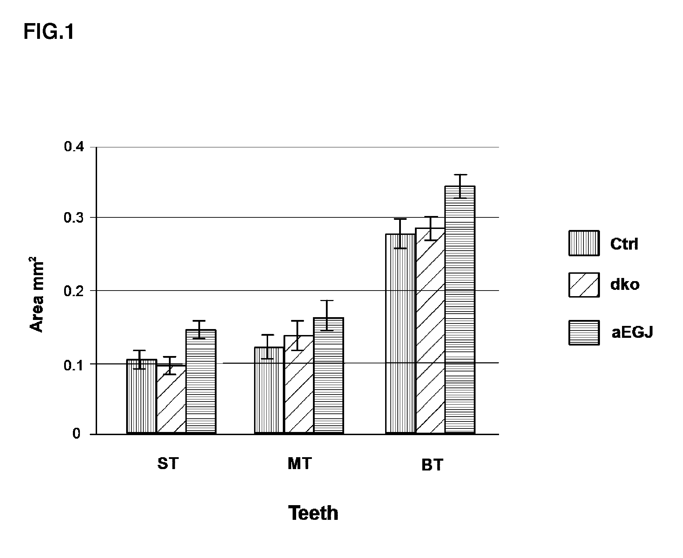

It was found that bone loss was significantly greater in the mice of non-treated control group as compared with that in the aCPT treated group (Table 1). Significant inflammatory response featured by inflammatory cell infiltration, edema and morphological abnormalities in periodontal tissues of the non-treated dKO mice was observed. However, the aCPT treated mice exhibited significantly mitigated inflammatory reaction with much less inflammatory cell infiltration and healthier morphological features. Most interestingly, more than half of the experimental animals in test group showed significant greater roots of molars with significant larger contact area with the alveolar bone, which was even greater than that in the control mice (wide type without dKO) at the same age (Fig. 1).

**Table 1. The aCPT treatment prevented the dental bone loss in dKO mice.**

| **Group** | | **pixels** | **Teeth area (mm²)** |
|---|---|---|---|
| **Crtl** | Small teeth | 2452.25 ± 261.5 | 0.102 ± 0.0135 |
| | Middle teeth | 3145.78 ± 578.9 | 0.131 ± 0.0239 |
| | Big teeth | 6729.80 ± 861.1 | 0.280 ± 0.0295 |
| **dko** | Small teeth | 2398.28 ± 404.5 | 0.100 ± 0.0160 |
| | Middle teeth | 3341.71 ± 685.1 | 0.138 ± 0.0275 |
| | Big teeth | 6775.33 ± 565.9 | 0.282 ± 0.0246 |
| **aCPT** | Small teeth | 3318.57 ± 961.5 | 0.145 ± 0.0280 |
| | middle teeth | 3957.87 ± 1586.7 | 0.156 ± 0.0315 |
| | Big teeth | 8060.16 ± 2033.3 | 0.336 ± 0.0461 |

| | | | |
|---|---|---|---|
| Ctrl: the wild type mice without dko; dko: the vehicle treated control group of mice with PS1 and PS2 genes conditionally knocked out in the forebrain; aCPT: the aCPT treated dko mice. | | | |

By contrast, none of the animals in non-treated control dKO mice showed the above mentioned results seen in aCPT treated dKO. Instead, their periodontal inflammation and the absorption of alveolar ridge was significant and the contact area of the molar roots with the alveolar bone was smaller compared with those in both the aCPT treated dKO mice and wild type control mice (without dKO) at the same age. The conclusion is that chewing food containing aCPT obtained from GJ could significantly prevent bone loss of molars and help maintain significantly larger contact area of the molars with the alveolar bone in dKO mice. Furthermore, the inflammatory process of the experimental periodontitis was also significantly mitigated by chewing aCPT containing food. In addition, significantly greater roots of molars were found in the aCPT treated dKO mice, even greater than those in the same age wild type control mice.

Example 3. The effects of aCPT on experimental periodontitis and aging oral condition in SAM mice.

The senescence-accelerated mouse (SAM) strain was established as a murine model of senescence acceleration and age-associated disorders (Takeda et al. 1981). In addition to the most characteristic age-related change in SAMP10, such as brain atrophy, these mice can be used as models of accelerated senescence in the study of dental disorders (Sashima et al., 1987; Chen et al.,1989). Human periodontitis and alveolar bone loss are considered as chronic diseases that become more prevalent and severe with advancing of age (Anderson, 1982). In this study, short lived mice (SAMP10) showing accelerated aging with a median survival time of 333 days were used (Takeda et al, 1991) and the aCPT was tested for its therapeutic effect on age associated oral disorders.

Methods. The SAMP10 mice (n=48) were maintained under conventional conditions on a chow diet and with tap water available at libitum. When the mice were 6 months old, the mice in test groups (n=8 each group) were fed with the conventional mouse chow containing 1% GJ, 1% EFRL, 1% ECA, 1% EUFRC or 1% ERI (i.e., aCPT made from five plants, respectively) for 3 months respectively. The mice in non-treated control group (n=8) were fed with conventional chow without any of these extracts. Three months after the treatment, these extracts containing mouse chows for feeding the mice in the five test groups were replaced respectively by the conventional chow without any of these extracts. When the mice were 10 months old, the gingival index (GI) (an assessment method based on visual inspection of gingivae to evaluate the color, the firmness and bleeding on probing of the gingival tissue) of mice in all groups was evaluated and the experimental mice were then sacrificed. The mean alveolar bone loss (mm SD) was evaluated by the sum of distances between the cusp tips and the alveolar bone along the axis of each molar root, subtracting from the contralateral side. Morphometric and histological analyses were performed in animals of all groups.

Results. Gingivitis scores (GI) were measured on a 0 to 3 scale system. Namely, 0 = No inflammation; 1 = Mild inflammation-slight change in color and little change in texture; 2 = Moderate inflammation-moderate glazing, redness, edema, hypertrophy and tendency to bleed upon probing; 3 = Severe inflammation-marked redness, hypertrophy and tendency to spontaneous bleeding. GI scores = sum of GI scores divided by the number of sites (gingival gum line around the tooth) scored.

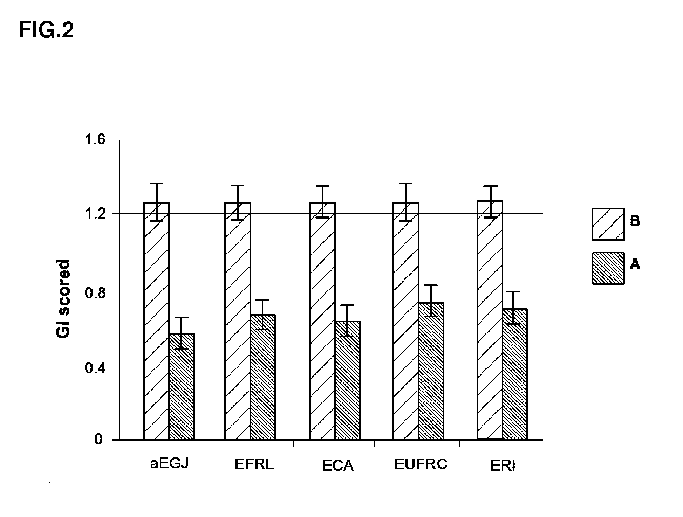

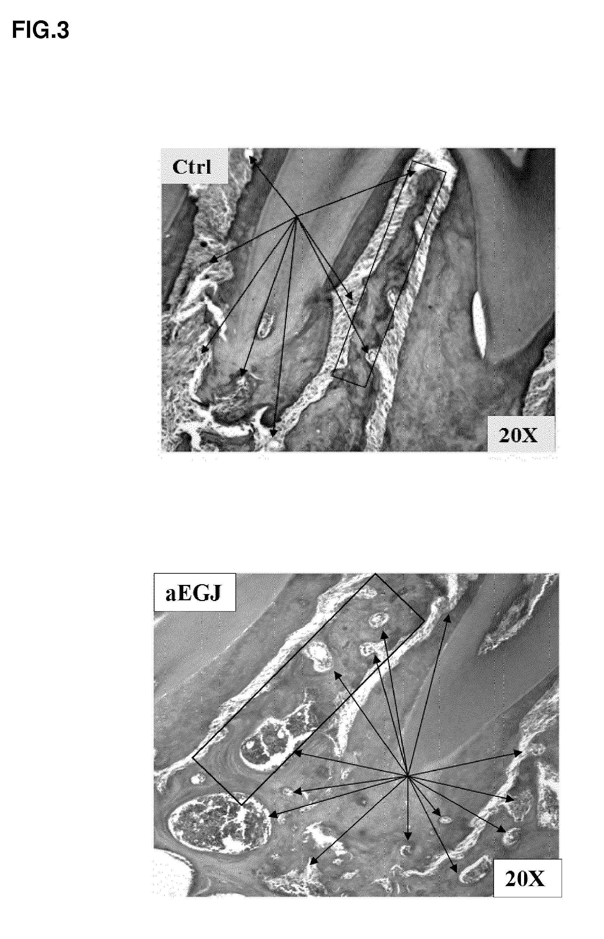

It was found that the GI scores of the GJ, EFRL, ECA, EUFRC or ERI respectively treated groups was significantly lower (0.601-0.732 ± 0.301) than that (1.232 ± 0.438) in non-treated control animals (Fig. 2) (p < 0.01). The alveolar bone loss was significantly prevented and the alveolar bone and cementum were well preserved in GJ and other extracts treated group as compared to that in non-treated control group (p < 0.05). Histological examination demonstrated that inflammation response, including edema and infiltration of inflammatory cells in periodontal tissue, and alveolar bone absorption were observed in non-treated control animals (Fig. 3). Furthermore, the firmness and the total volume of the periodontal tissue of non-treated control animals were significantly reduced. By contrast, in the GJ and other extract-treated animals, the inflammatory reaction was significantly reduced with significantly less edema and inflammatory cell infiltration. Furthermore, significantly more (9.3-11.2%) capillaries were observed (p < 0.05) in the whole periodontal tissue (Fig. 3) with significantly greater volume and firmness of the periodontal tissue as featured by more fibroblasts and deposited collagens.

In sum, these observations imply that all five extracts, especially GJ, not only inhibited the inflammatory response, including edema and inflammatory cell infiltration, of the periodontal tissues and alveolar bone absorption in aging mice, but also stimulated the substantial growth and repair of periodontal tissues in the teeth gum, resulting in significantly improved gingivitis, periodontitis and gingival atrophy. In summary, the extracts including GJ, EFRL, ECA, EUFRC and ERI can significantly reduce the progress of aging-associated periodontal inflammation response, gingivitis, gingival atrophy and alveolar bone absorption.

### Example 4. The effect of gum strengthening and restoration of gum recession

Tooth crown is the upper portion of the tooth that is above the alveolar bone. Tooth root refers to the lower part of the tooth in the alveolar bone. The desirable crown-to-root-ratio is between 1:1 to 1:2 as a critical index in the prognosis of a tooth and its gum (Czochrowska et al., 2002). To evaluate the effect of aCPT on gum regeneration, we computed crown-to-root ratio for both control (SAMP10, n=8, 6 month old) mice, which were fed with conventional mice chewing chow, and treated SAMP10 mice (n=8, 6 month old), which were fed with 1% aCPT or GJ containing chewing chow. After 3 months feeding, poor crown-to-root-ratios of molar teeth were found in control mice with more root portion of the molars exposed. By contrast, the crown-to-root-ratios of molar teeth in the 1% aCPT or GJ treated mice appeared normal with significantly less exposed root portion of their molars.

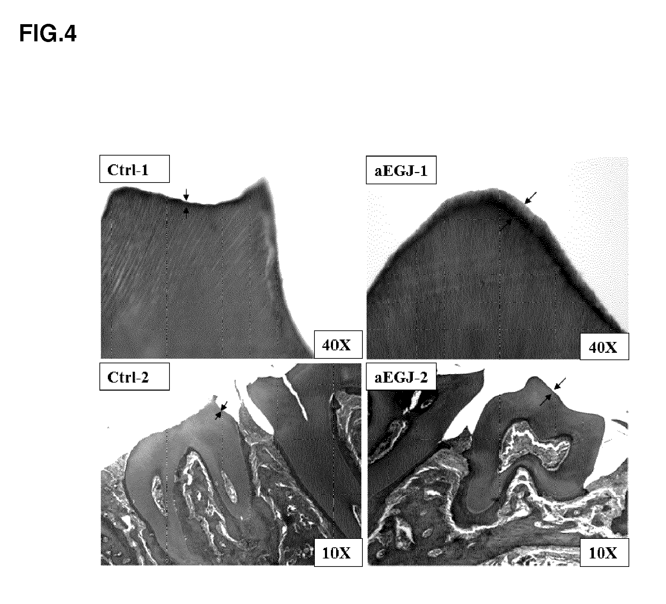

In human beings, the thickness of the tooth enamel begins to decrease at about age 50. However, in the present study with SAMP10 mice, it was also found that at 9 months old, the enamel thickness of the SAMP10 mice in the vehicle treated control group significantly decreased and the dimensions of the enamel on the surface of the teeth were less continuous (Fig. 4). By contrast, both the enamel thickness and dimensions of the mice in aCPT or GJ treated group were preserved (Fig. 4) indicating aCPT or GJ treatment can probably prevent the degenerative changes of the enamel due to chronologic age (P<.001).

Loose Teeth. If one is aware of looseness of some teeth, this is a very clear sign that advanced gum disease may be present. When the patient is aware of looseness, it is usually a very bad sign since patients do not perceive looseness until the teeth are very loose and sometimes hopeless.

Gum disease is often caused by various types of bacteria that are normally in the mouth. Some patients may have more types of bacteria in their mouth that are associated with aggressive gum disease. The bacteria easily accumulate at the junction where the gums meet the teeth. Normal healthy gums have a ditch or sulcus (1-3 millimeters depth) surrounding the teeth. When the bacteria infect the gums, the gums may detach from the teeth. When the gums detach from the teeth as a result of the gum disease, the ditch forms a "pocket", which has a depth deeper than 3 millimeters. The bacteria that cause gum disease may then spread into the underlying bone which supports the teeth, consequently causing loosening of the teeth. If the gum diseases are not treated properly at their early stages, the patients may lose their teeth.

We have also tested the therapeutic effect of GJ (aCPT of GJ) containing toothpaste (CPT-T, formulation are described above) on oral diseases in human beings, the subjects (n=12), nine with mild gum recession (40-60 years old), two with loose teeth (52-70 years old) and one with severe gum recession and loose teeth (46 years old) were subject to application of CPT-T (3%) twice a day. On examination prior to the use of CPT-T, all subjects were found having larger gaps in between their teeth indicating overall gum recession. In addition to the widened gaps between the teeth, the two subjects with loose teeth (1-3) in their lower frontal (left) and upper frontal (right) teeth showed a dark-brown color of these loose tooth for more than 2-3 years. The subject having severe loose teeth of her all teeth showed a dark-brown color of all her teeth with biting ability severely reduced. On further examination, it was found that more than half of her tooth roots (front, upper, lower and rear teeth) were exposed due to receding gums and her gums were easily bleeding upon probing or brushing with bad breath indicating gingivitis and gum receding.

These test subjects were taught to brush teeth for at least 3 minutes twice daily in the morning and at night with CPT-T (3%, 3 grams aCPT of GJ in 97 grams toothpaste). The results showed that after 2-3 weeks application of CPT-T, all subjects reported that they felt that the occasions that pieces of hard food or food fibers were wedged between the teeth were significantly reduced after CPT or GJ application, indicating that the space between the teeth was reduced probably due to strengthened gums that can hold the teeth in positions tighter. Upon examination, the gaps between teeth in all subjects became approximately 1/3 smaller than that prior to application of CPT-T. The loose teeth of two subjects were less removable with the dark-brown color of the affected teeth significantly lightened. The redness and tenderness of the gum in most of the subjects turned to pink and significantly improved. The bad breath of the mouth had gone probably due to the significantly improved mouth bacterial infection and inflammation.

After six weeks application of CPT-T, it was found that the already reduced space of the teeth in all subjects had been maintained. In general, the teeth appeared whiter and more solid after using CPT-T. On examination, the loose teeth of two subjects became complete normal and the previous dark-brown color of these loose teeth due to ill-nourishment turned to normal white color as all other normal teeth. The redness and tenderness of the gum in some subjects turned to a normal coral pink color and the infection and inflammation had all gone. The bad breath due to mouth bacterial infection, inflammation and oral diseases had significantly improved or completely disappeared. After six weeks of application of CPT-T, the subject with severe gum recession and loose teeth (46 years old) was also significantly improved in that all her loose teeth became less movable and the redness, tenderness and gum bleeding of her gums mitigated significantly with her chewing ability restored. However, she may need longer period and higher concentration of CPT-T to restore her receded gums and loose teeth. All above mentioned results indicate the strengthening of the receding gums holds the swing teeth tighter and provides good nourishment to the teeth. More interestingly, the previously exposed areas of teeth roots in some of these subjects with gum recession were found smaller and better covered after two moth use of CPT-T. It was also found from our other tests that higher concentration of aCPT, such as 9-25% GJ toothpaste, contained in the toothpaste tend to deliver better therapeutic effects on oral diseases.

Looseness of some teeth is a clear sign that advanced gum disease may be present. Normally the loose teeth are clinically hopeless and the patient with loose teeth will probably lose their teeth. Since CPT-T can significantly inhibit the growth of pathogenic microbes and promote tissue repair, it can remove the direct causal microbes of gum diseases and enhance repair of the damaged gum. As a result CPT-T can prevent and treat gum diseases and gum recession as demonstrated above.

### Example 5. Effects of CPT-T on gum bleeding.

CPT-Ts (0.1%, 0.5% or 3% containing 0.1, 0.5 or 3 grams GJ per 100 gram toothpaste respectively) was tested on 9 individuals (ages ranging from 26 to 80) with either acute or chronic gum bleeding in each group. It was found that brushing with CPT-T (∼1g) for 2 minutes, once in the morning and once at night, stopped gum bleeding in all 9 individuals. Depending on the severity of gum bleeding, the time length required to stop gum bleeding varied from 1-6 days. In 2 cases, gum bleeding was disappeared after 1 day of CPT-T brushing. In another 4 cases, gum bleeding was stopped after 3-5 days of CPT-T treatment. Two weeks of brushing were long enough to stop the gum bleeding in the remaining 3 cases who had reported having recurrent intractable gum bleeding in the last 2-3 years. By contrast, the gum bleeding in only one case of the 9 control subjects, who were using toothpaste without containing aCPT, stopped gum bleeding after 6 days brushing.

Interdental Bleeding Index (IBI) measures the number of interdental spaces that bleed, which is divided by number of interdental spaces studied to yield a score with a minimum of 0 (no bleeding) and a maximum of 1 (bleeding). The numbers of bleeding spots are divided by number of spots between teeth that are scored. The IBIs are 0.387 ± 0.286 in placebo comparator (n=9) and 0.365 ± 0.276 in aCPT group (n=9) respectively prior to the use of the toothpastes. However, after two weeks uses of the toothpastes, the IBIs are averagely 0.358 ± 0.266 in placebo comparator who were using toothpaste without containing aCPT, and 0.101 ± 0.098 in aCPT group who were using toothpaste containing 1-3% aCPT, respectively.

### Example 6. The rapid therapeutic effects of CPT-T on mouth ulcers

Six individuals (4 females and 2 male, ages ranging from 48 to 82), who had suffered from repeated mouth ulcer, were subject to use CPT-T (1% GJ) to brush teeth for at least 3 minutes twice daily, one in the morning and one at night. In all six subjects, mouth ulcers were completely disappeared after 1-7 days CPT-T treatment. The duration for healing the mouth ulcer varied from 1 day to 1 week. Depending on the severity of the ulcer and the concentration of the aCPT contained in the toothpaste, the ulcer could be healed within 1-3 days in subjects suffering mild or accidental ulcer cases, however, the heal of ulcer may require 6-8 days for the refractory mouth ulcer. Normally the higher the concentration of aCPT contained in the toothpaste, the sooner the mouth ulcer healed. For example, one female who had suffered recurrent and refractory mouth ulcer for more than two years, and her incurable ulcer was cured after 7 days application of CPT-T (3% GJ). By contrast, in the control group, 3 subjects with accidental mouth ulcer were healed on day 4, 5 and 8 after using control toothpaste. However, none of the other 3 subjects with recurrent mouth ulcer showed any improvement of their ulcers.

### Example 7. Effects of CPT-T on anti-mouth bacteria and prevention of tooth decay

The mouth is a complimentary medium for flourishing of a variety of bacteria, including mainly streptococci, lactobacilli, staphylococci, corynebacteria and a great number of anaerobes, due to the existence of nutrients, cell debris and emissions. Dental caries is mainly caused by acidic metabolites of bacteria, such as Lactobacillus spp., Streptococcus mutans and Actinomyces spp. These bacteria usually live in the mouth and nourish on carbohydrates. These bacteria form sticky and slimy bio-films coating in the mouth and on the teeth known as bacterial plaque. Bacterial plaque is most visible in the morning before brushing. These bacteria contained in the clear, sticky and slimy film, covering on the teeth and gums metabolize mainly sugar contained in the eating food through anaerobic respiration, resulting in the production of sour bacterial wastes creating a high level of acidity on the surface of tooth. The acids attack the teeth, destroy tooth enamel and reduce the mineral content of teeth, resulting in tooth decay over a period of time.

Inhibiting effect of aCPT on the binding of bacteria to the teeth surface. Oral bacteria adhere to tooth surface through carbohydrate binding proteins, while the polyphenols contained in the aCPT can precipitate carbohydrate binding proteins of the bacteria. Therefore aCPT can inhibit the binding of bacteria to the tooth which would result in decreased adherence. It was shown that 0.3% aCPT was capable of reducing the adherence of Streptococcus mutans to the tooth surface.

Inhibiting effect of aCPT on bacterial growth. In this study the bacterial growth inhibiting effects of aCPT was tested. Commercially available preservatives widely used in toothpastes, shampoo, cosmetics and body products, such as 0.2% benzoic acid, 0.2% chlorhexidine, 0.4% parabens, 0.3% triclosan, 0.15% potassium sorbate, were used as positive controls.

Dental toothpicks were used to collect the oral bacterial samples taken from the gap between the teeth from 8 normal subjects (4 male and 4 female). Human oral saliva in the mouth is composed of water, amino acids, proteins, lipids, carbohydrates and some inorganic substances, thus it is suitable for bacteria growth. The bacterial plaque formed on tooth surface and gums contain mainly the anaerobic spirochete bacteria and vibrio (comma-shaped bacteria), Staphylococcus, Lactobacillus, streptococci (particularly Streptococcus mutans) and Actinomyces odontolyticus.

This experiment was designed to investigate the inhibiting effect of aCPT on the growth of oral bacteria. The sample toothpicks were inoculated by aseptically streaking on the mannitol salt agar (MSA) plates respectively. The inoculated plates were cultured at 37°C.

The MSA plates contained each of aforementioned preservatives at the concentrations (0.2% chlorhexidine, 0.2% benzoic acid, 0.4% parabens, 0.3% triclosan, 0.15% potassium sorbate) according to the recommendations of national regulation authority as positive controls. The maximal inhibiting dilution (MID) of aCPT was measured in batch cultures for each culture testing subject.

All these five preservatives have been reported for their effects on plaque prevention and inhibition of bacteria. Therefore, it is the aim of this study to compare the effects of these five commercially available preservatives with aCPT at different concentrations on the growth of oral bacteria, which play an important role in the occurrence of dental caries and gingivitis.

The cultures were incubated in an atmosphere of 80 per cent CO2, 15 per cent N2, and 5 per cent H2. All sample cultures were cultured to reach the densities between 0.5 x 10⁹ and 1 x 10⁹ cells/ml, which were used to inoculate second cultures containing respective preservatives or various concentrations of aCPT. Growth inhibiting effects of the 5 preservatives and aCPT for the oral bacteria obtained from different subjects were determined by counting the bacterial colonies of the culture plates respectively.

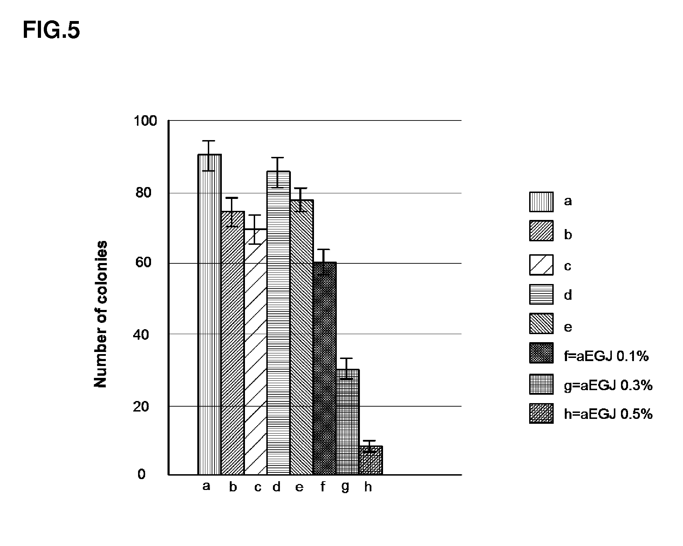

It was shown that the oral bacterial colonies were observed everywhere in the whole field of the culture plates with the aforementioned 5 preservatives at the State recommended concentrations (Fig. 5). By contrast, although there were similar numbers of bacterial colonies of aCPT treated cultures observed at the concentration of 0.1% aCPT or GJ as observed in preservative-treated oral bacteria cultures, there were approximately 65% less bacterial colonies found once aCPT or GJ concentration reached 0.3% (Fig. 5). When 0.5% aCPT or GJ was used to treat the culture, only less than 10 bacterial colonies were observed (Fig. 5). There were almost no or less than 5 bacterial colonies observed when the concentration of aCPT or GJ was 1% or higher.

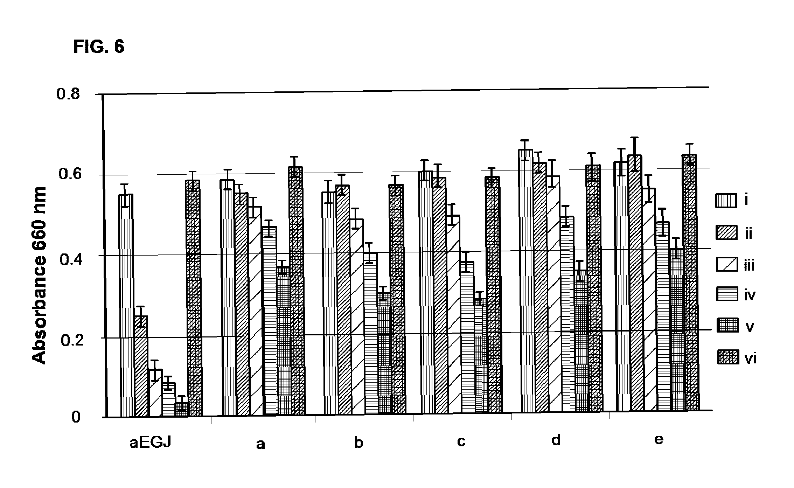

We then tested the growth inhibiting effect of aCPT on the oral bacteria obtained from a human subject with oral inflammation in liquid bacterial culture. The subject who was suffering oral inflammation brushed his teeth at night and rinsed his mouth in the next morning with 1640 medium containing 10% FBS. This oral rinse was transferred into 24 well culture plates and every well contained 1 ml of the rinse. Various concentraions of the 5 aforementioned preservatives and aCPT were added to the wells in three parallel holes respectively and the plates were incubated at 37°C incubator. After 18 hours culture at 37°C, a dose dependent inhibiting effect on the growth of sampled oral bacteria by aCPT (0.1%, 0.2%, 0.4%, 0.8% and 1.6%) was observed. By contrast, although a weak dose dependent inhibiting effect on the growth of oral bacteria by these selected preservatives (0.05-0.3% chlorhexidine; 0.05-0.3% benzoic acid; 0.1-0.5% parabens; 0.05-0.4% triclosan and 0.05-0.2% potassium sorbate) was observed, the potency of the inhibiting effect of these preservatives is weak with only 15-35% growth inhibiting effect at the national recommended concentrations (Fig. 6). In a sharp contrast, although 0.1% aCPT or GJ treated bacterial culture showed about 10% reduction of the growth of the bacteria in the culture that is equivalent to the selected preservatives at the State recommended concentration, 0.2-1.6% aCPT or GJ inhibited the growth of the bacteria by 60% (0.2%), 81% (0.4%), 90% (0.8%) and 96% (1.6%) respectively (Fig. 6). These results indicate the superior inhibiting effect of aCPT on the growth of human oral bacteria over that of the 5 widely used preservatives.

It is well-known that preservatives can cause various side effects such as skin irritations, carcinogenic and nerve system and etc, as a result, in 2009, the new national standard has been included to allow the addition of preservatives no more than 0.3%. Therefore, a more active preservative at low concentration would be preferred. Since aCPT was fractioned from a natural edible plants (Gj or EFRL or other plants) and equivalently active at lower concentrations (0.1-0.2%) compared to that of above widely used preservatives at their recommended concentrations, therefore aCPT is considered as a safer green preservative with equivalent anti-oral bacterial effect at 1/2-1/3 lower dose than the national recommended concentrations of the tested widely used preservatives. Furthermore, when the concentration of aCPT increased to 2/3 or equivalent concentration of the nation recommended concentrations of these preservatives, its inhibiting effect on oral bacterial growth is 1.7-4 or 2.3-5.4 folds higher compared to those of the preservatives tested at their national recommended concentrations (Fig. 6). These results suggest a much more potent inhibiting effect of aCPT on the growth of human oral bacteria and its better safety compared with that of the 5 widely used preservatives. More importantly, since the natural composition of the edible plant is not changed, therefore, 0.1-1.6% concentration of the active fraction containing hundreds of different natural compounds is equivalent to hundreds folds less concentrations for each compound. Moreover, any possible toxicity test demonstrated that oral administration of aCPT 480-4800mg/kg weight of rats daily for 60 days, which is 1,600,000-16,000,000 or 400,000-4,000,000 folds higher than the dose for 0.5-2% aCPT toothpaste , did not produce any obvious side effect to the experimental animals. In addition, before we made the patent application, we have tested the toothpaste containing 0.1-28% aCPT respectively for more than two years, except for its potent multi-therapeutic effects on oral diseases, no any undesired or side effects have been observed. Therefore, aCPT toothpaste is not only able to replace the less effective and possible side effects proned synthesized preservatives, but also provide extraordinary therapeutic effects over variety of oral diseases due to the nature of aCPT containing natural anti-bacterial factor(s), anti-inflammation factor(s), promoting regeneration of gum tissues in aging gums and oral tissues, and tissue repairing factor(s) that can induce stem cell or progenitor cells to differentiate into local tissues to repair damaged or recessed gum and oral tissues.

### Example 8. The effect of reducing and preventing bacterial plaque formation on the teeth

The demonstration of bacterial growth inhibiting effect of aCPT in vitro studies predicts the potential of its possible anti-bacterial plaque formation on the surface of teeth. To test this, 16 human subjects were recruited and subject to removing the tartar and polishing the teeth using rubber wheel prior to the trial as the experimental baseline. All subjects were subject to the check of their gums and teeth prior to, during (one week and one month since the use of the toothpaste) and after (three months after the use of the toothpaste) the use of the CPT-T. At least 6 points of each tooth were checked. Since the speedy effect of CPT-T on the removal of bacterial plaque and inhibition of the plaque formation on the teeth, after one week of the use of CPT-T, the evaluation of the effectiveness of the CPT-T in removal of plaque and anti-bacterial plaque formation were performed.

The evaluation of plaque index (PLI) is on a scale 0 to 5 (0 = no plaque, 1 = separate flecks of plaque on the tooth, 2 = a thin continuous band of plaque, 3 = a band of plaque up to one-third of the tooth, 4 = plaque covering up to two thirds of the of the tooth, 5 = plaque covering two-thirds or more of the crown of the tooth. Plaque score=sum of all scores divided by the number of sites (teeth) scored.

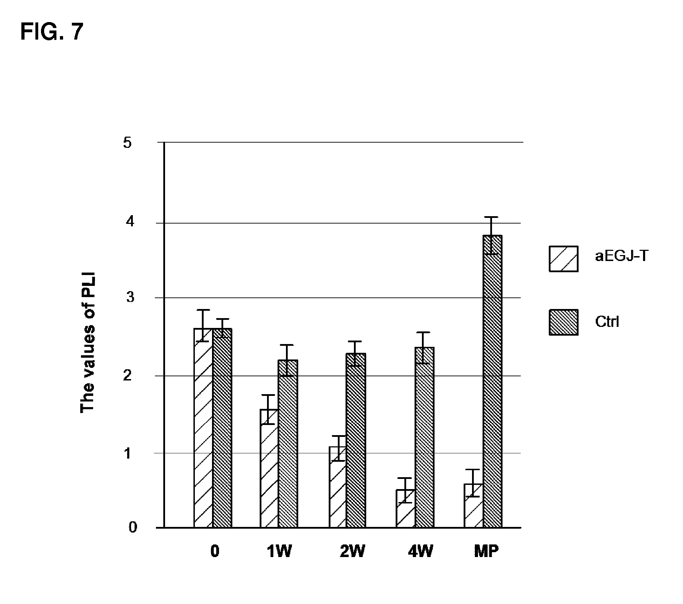

The human subjects in test group (n=30, ages ranging from 26 to 62) used CPT-T (1-3% GJ) while the subjects in control group (n=30, ages ranging from 24 to 58) used the same toothpaste without containing aCPT to brush teeth for at least 3 minutes twice daily, one in the morning and one at night. The evaluation of PLI in all subjects in both groups was performed prior to application of the test toothpastes and the results show that the average PLI scores are about 2.61±1.08 (test group) and 2.59 ±1.19 (control group) respectively (Fig. 7). However, with the application of CPT-T, the PLI scores in the test group were progressively decreased (1.61 ± 0.82 at week 1, 1.03 ± 0.65 at week 2, 0.51 ± 0.31 at week 4 post application of CPT-T) (p < 0.001) (Fig. 7). By contrast, the PLI scores in control group did not significantly changed (2.53 ± 1.21 at week 1, 2.32 ± 1.41 at week 2, 2.36 ± 1.12 at week 4 post application of the control toothpaste (Fig. 7). More significantly, after two weeks application of the toothpastes, when the subjects in both groups were checked in the morning before teeth brushing, the surfaces of the teeth in the test group remained smooth with no obvious bacterial film formed and the PLI was about 1.13 ± 0.61. In a sharp contrast, it was found that the surfaces of the teeth in the subjects of control group were covered with a thin and rough film of bacteria and the PLI was about 3.86 ± 1.65 (p < 0.001) (Fig. 7). This thin and rough dental film contains many microbes including mutans streptococci and several other bacteria that ferment sugars and other carbohydrates to form lactic and other acids. Repeated cycles of acid generation can gradually dissolve the underlying mineral, both at the surface and at the subsurface, which causes irreversible structural lesion in the dental hard tissue with the presentation of an opaque white or brown spot under the enamel surface, and consequently dental caries. Hence, the bacterial growth inhibiting effect of CPT-T and consequently the inhibition of dental bacterial plaque formation on the surface of teeth would provide strong protection of teeth from bad breath, dental caries and oral inflammation. More interestingly, after 1-2 months application of CPT-T in four subjects from the test group who had several rough and opaque white spots on the occlusal surface of their teeth formed due to the dissolution of the minerals in their teeth enamel, the rough and porous part of the occlusal surface of the teeth were enameled to smooth surface with the opaque color restored to a normal appearance indicating the enamel-remineralisation and restoration of the tooth decay at their early stage. The newly repaired surface of the lesion appeared hard and shiny, in contrast to the chalky, rough, and porous surface of the active lesions in other three subjects from control group.

### Equivalents of the plant extract

According the present invention, the ethanol extracts of the five different plant species, fructuo rosae laevigakea (EFRL), Centella asiatica (ECA), unripe fruits of Rubus coreanus (EUFRC), Geum japonicum (GJ), and Rubus imperialis (ERI), contain similar compositions of active compounds from each other. They can be considered as equivalents to each other in use. The equivalence was demonstrated by the experiments in Example 3, where the extracts made from each of the equivalent plants using the same extracting method described in the Example 1 (refereed to as EFRL, ECA, EUFRC, GJ and ERI, respectively). It was found that the EFRL, ECA, EUFRC, GJ and ERI processes similar effects on repair of gum recession, mouth damage, gum ulcer, bleeding gum and mouth inflammation. An exemplified data is shown in FIG. 2. Therefore, for all purposes and intentions, they are considered equivalents to each other in practicing the present invention.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being defined by the following claims.

## Claims

1. An oral healthcare product comprising an effective amount of an active plant extract containing chlorophyll, polyphenols and triterpenoids as well as other unknown compounds ("aCPT") in an orally acceptable dosage form and a product indication indicating said product is for improving oral health.

2. The product of claim 1, wherein said orally acceptable dosage form is selected from the group consisting of a cream, an ointment, a gel, a foam, a powder, a solution, a spray, a candy, a lozenge, a toothpaste, a gargle, and a gum.

3. The product of claim 1, wherein the orally acceptable dosage form is selected from the group consisting of mouth wash, mouth rinse, oral care gel strips, and dental floss.

4. The product of claim 2, wherein the orally acceptable dosage form is a toothpaste.

5. The product of claim 1 wherein said aCPT is made from a plant species selected from the group consisting of Fructuo rosae laevigakea (EFRL), Centella asiatica (ECA), unripe fruits of Rubus coreanus (EUFRC), Geum japonicum (GJ) and Rubus imperialis (ERI).

6. The product of claim 5, wherein said orally acceptable dosage form is selected from the group consisting of a cream, an ointment, a gel, a foam, a powder, a solution, a spray, a candy, a lozenge, a mouthwash, a toothpaste, a gargle, and a gum.

7. The product of claim 5, wherein the orally acceptable dosage form is selected from the group consisting of mouth wash, mouth rinse, oral care gel strips, and dental floss.

8. The product of claim 7, wherein the orally acceptable dosage form is a toothpaste.

9. A healthcare product comprising an extract of a plant specie selected from the group consisting of Fructuo rosae laevigakea (EFRL), Centella asiatica (ECA), unripe fruits of Rubus coreanus (EUFRC), Geum japonicum (GJ), and Rubus imperialis (ERI) for use in a method for improving oral health by administering to a person.

10. The healthcare product of claim 9, wherein said healthcare product in the form of toothpaste.

11. The product of claim 5, wherein said aCPT is made from Geum japonicum.

12. The product of claim 5, wherein said aCPT is made from rosae laevigakea.
